# EUROPEAN PATENT APPLICATION

(11) **EP 4 631 418 A1**
(43) Date of publication of application: **15.10.2025**
(21) Application number: 24169844.8
(22) Date of filing: 12.04.2024
(51) Int. Cl.: A61B 5/00, A61B 6/00, A61B 8/00

(54) **ALIGNMENT OF MEDICAL DATA ACQUISITION**

(71) Applicant: Koninklijke Philips N.V., 5656 AG Eindhoven (NL)
(72) Inventor: ELENBAAS, Thijs, Eindhoven (NL); HENDRIKS, Bernardus Hendrikus Wilhelmus, Eindhoven (NL); OLIVAN BESCOS, Javier, 5656AG Eindhoven (NL); ADMIRAAL, Alexander Johannes, Eindhoven (NL); GRANDIA, Leo, Eindhoven (NL); BACHVAROV, Chavdar Ludmilov, Eindhoven (NL)
(74) Representative: Philips Intellectual Property & Standards

(57) **Abstract**

The present invention relates to data acquisition in a multi-modality arrangement. In order to provide a facilitated way of combining different data sources, a multi-domain signal generator (10) for temporal alignment of medical data acquisition in a multi-modality arrangement is provided. The generator comprises an activation interface (12), a control circuit (14) and a timestamping provider (16). The activation interface is configured to receive an input signal (18) for a temporal alignment of the multi-modality arrangement and to transmit the input signal to the control circuit. The control circuit is configured to provide a trigger signal (20) for an activation of the timestamping provider upon receiving the input signal. The timestamping provider is configured, upon receiving the trigger signal, to activate a generation of a predetermined multi-modality timestamp pulse (22); wherein the timestamp pulse causes a modulation of at least one physical parameter of a first measuring signal (24) receivable by a first medical data acquisition modality (26) and a modulation of at least one physical parameter of a second measuring signal (28) receivable by a second medical data acquisition modality (30), the first measuring signal and the second measuring signal being from different medical data acquisition modalities.

## Description

### FIELD OF THE INVENTION

The present invention relates to data acquisition in a multi-modality arrangement. The present invention relates in particular to a multi-domain signal generator for temporal alignment of medical data acquisition in a multi-modality arrangement, to a multi-modality medical data acquisition system and to a method for temporal alignment of medical data acquisition in a multi-modality arrangement.

### BACKGROUND OF THE INVENTION

In medical environments like examination or operation rooms in a hospital, different types of data may be acquired during a medical procedure. In order to make enhanced use of the data, different combinations of data can be required. For correlation purposes, synchronization of different modalities can be necessary. However, it has been shown that this may require control interfaces of the different modalities and can be cumbersome in view of required flexibility within the operation room.

### SUMMARY OF THE INVENTION

There may thus be a need to provide a facilitated way of combining different data sources in a medical environment.

The object of the present invention is solved by the subject-matter of the independent claims; further embodiments are incorporated in the dependent claims. It should be noted that the following described aspects of the invention apply also for the multi-domain signal generator, for the multi-modality medical data acquisition system and for the method for temporal alignment of medical data acquisition in a multi-modality arrangement.

According to the present invention, a multi-domain signal generator for temporal alignment of medical data acquisition in a multi-modality arrangement is provided. The generator comprises an activation interface, a control circuit and a timestamping provider. The activation interface is configured to receive an input signal for a temporal alignment of the multi-modality arrangement and to transmit the input signal to the control circuit. The control circuit is configured to provide a trigger signal for an activation of the timestamping provider upon receiving the input signal. The timestamping provider is configured, upon receiving the trigger signal, to activate a generation of a predetermined multi-modality timestamp pulse. The timestamp pulse causes a modulation of at least one physical parameter of a first measuring signal receivable by a first medical data acquisition modality and a modulation of at least one physical parameter of a second measuring signal receivable by a second medical data acquisition modality. The first measuring signal and the second measuring signal are from different medical data acquisition modalities.

As an effect, the modulation of the first acquired data and the modulation of the second acquired data can be used for synchronization of the first acquired data and the second acquired data.

As an advantage, multi-modality registration, as a very challenging task that is vital for combining multiple modalities, is facilitated. The combining conveys a maximum amount of data while requiring minimal interpretation by the physician. This is suitable in view of technological advances and experience of physicians in minimally invasive interventions that have resulted in a gradual increase in complex procedures where the use of multiple modalities is wanted. When the anatomy of interest changes during the acquisition of the modalities, temporal registration is needed. The multi-domain signal generator addresses this.

While temporal registration is very important but may be difficult to achieve, a field of application for the present solution is modalities that show vastly different properties.

According to an example, the timestamp pulse is provided to modify at least one control parameter that has an influence on the measuring signal of at least two different data acquisition modalities.

According to an example, the timestamping provider comprises at least one interface for providing a data connection with a signal generator that provides at least one of the first and second measuring signal. The timestamp pulse leads to a modification of the measuring signal provided by the signal generator.

According to an example, the timestamping provider comprises at least one pulse generator that provides a first signal pulse in a domain of the first measuring signal, and a second signal pulse in a domain of the second measuring signal.

According to an example, the first measuring signal and the second measuring signal are from different signal domains.

According to the present invention, also a multi-modality medical data acquisition system is provided. The system comprises a plurality of data acquisition modalities comprising at least a first medical data acquisition modality applying a first measuring signal to provide a first data output and a second medical data acquisition modality applying a second measuring signal to provide a second data output. The first measuring signal and the second measuring signal are different from each other. Further, the system comprises a multi-domain signal generator according to one of the preceding examples. The multi-domain signal generator generates a signal that is received by several data acquisition modalities as a timestamp for temporal alignment.

According to an example, the timestamp pulse causes i) a modulation of at least one physical parameter of the first measuring signal received by the first medical data acquisition modality. The timestamp pulse also causes ii) a modulation of at least one physical parameter of the second measuring signal received by the second medical data acquisition modality.

According to an example, the modulation of the at least one physical parameter of the first measuring signal provides a first timestamp signal as part of the first data output. The modulation of the at least one physical parameter of the second measuring signal provides a second timestamp signal as part of the second data output.

According to an example, further medical data acquisition modalities are provided applying measuring signals to provide a plurality of data outputs. The timestamp pulse causes a modulation of at least one physical parameter of each of the measuring signals received by the further medical data acquisition modalities.

According to an example, one of the data acquisition modalities is an X-ray imaging modality comprising an X-ray source and an X-ray detector. The multi-domain signal generator comprises an X-ray source. Further, the multi-domain signal generator is arranged near the X-ray detector of the X-ray imaging modality.

According to an example, one of the data acquisition modalities is an ultrasound imaging modality. The multi-domain signal generator comprises a sound source to generate a sound signal receivable by the ultrasound imaging modality.

According to an example, one of the data acquisition modalities is an imaging modality based on light. The multi-domain signal generator comprises a light source to generate a light signal receivable by the ultrasound imaging modality.

According to an example, one of the data acquisition modalities is provided as at least one of the group of medical imaging comprising X-ray, CT, ultrasound, EM imaging or MRI, and patient related data comprising hemodynamic, ECG and other vital data, and device tracking comprising shape tracking, and spatial and environmental imaging comprising time of flight, LIDAR, and optical imaging comprising staff videos, and acoustic recording comprising staff communication and procedure recording, and radiation tracking.

According to an example, based on the timestamp pulse, data acquired by the different data acquisition modalities is combinable in a temporally synchronized manner.

In an option, the timestamp pulse is provided in a resolution according to a smallest resolution of the different data acquisition modalities.

According to the present invention also a method for temporal alignment of medical data acquisition in a multi-modality arrangement is provided. The method comprises the following steps:
- Acquiring medical data with a plurality of data acquisition modalities comprising at least a first medical data acquisition modality applying a first measuring signal to provide a first data output and a second medical data acquisition modality applying a second measuring signal to provide a second data output;
- Receiving an input signal for a temporal alignment of the multi-modality arrangement;
- Providing a trigger signal for an activation of the timestamping provider upon receiving the input signal; and
- Generating a predetermined multi-modality timestamp pulse; the timestamp pulse causes a modulation of at least one physical parameter of the first measuring signal receivable by the first medical data acquisition modality and a modulation of at least one physical parameter of the second measuring signal receivable by the second medical data acquisition modality, the first measuring signal and the second measuring signal being from different medical data acquisition modalities.

According to an aspect, a multi-modal synchronizer event e.g. "clapper board" is provided. A synchronized timestamp is injected in multiple modalities, e.g. functioning as a t=0 event that makes it easy to synchronize modalities.

Instead of adding a separate timestamp signal in a separate domain to the respective acquired medical data, the timestamping provider generates a signal such that the timestamp event can be identified within the acquired medical data. In other words, the timestamping is provided within the respective domain of the acquired medical data. The timestamping is integrated into the recorded data. A time-related signal portion is implemented into the detected signal of the medical data. This allows to provide a time-stamping and thus synchronizing of different types of data even when an interface and thus access to a third party's medical data acquisition modality is not given.

The timestamp signal for temporal alignment is integrated into the acquired medical data in an identifiable way. The timestamp signal is so-to-speak infused into the acquired data.

According to an aspect, the timestamp signal is provided such that it can be recorded within the respective domain of different modalities in a multi-modality arrangement.

According to an aspect, the multi-domain signal generator generates a signal that is received by several data acquisition modalities, such as different medical imaging modalities. For this purpose, the multi-domain signal generator generates a multi-domain signal. In a first option, the multi-domain signal provides a modulation of a physical parameter of a respective measuring signal and the modulation is detected by the respective measuring signal receiving or detecting part of the data acquisition modality. The timestamp pulse is thus directly provided to the detecting side of the data acquisition modality where it becomes integrated into the acquired data set. In an option, the multi-domain signal. In a second option, the multi-domain signal provides a triggering of a modulation of the respective measuring signal and the modulation is detected by the respective measuring signal receiving or detecting part of the data acquisition modality. Via the generated measuring signal, the timestamp pulse is thus indirectly provided to the detecting side of the data acquisition modality where it becomes integrated into the acquired data set.

As an example, temporal registration for intra-procedural guidance as well as data mining is provided.

This is of advantage, for example, in modalities that show vastly different properties.

The present solution is facilitated over correlating visual changes in the modality, e.g. the cardiac heart phase, diaphragm movement, device movement. The integrated timestamp is also suitable for both spatial and temporal changes which adds another dimension to the solution space, otherwise requiring more computational effort and lowering chance of successful alignment. The timestamping addresses large temporal offsets, which would otherwise render algorithm-based registration very costly: Where spatial offsets may have a relatively small range of scale, e.g. from offsets in centimeters, and a required accuracy in millimeters, temporal offsets may have a much larger range of scales: the offset may be in the scale of hours, while at the same time sub-second accuracy is needed. The timestamping also addresses that cyclic movements can easily result in incorrect phase swifts, e.g. heart cycle, breathing cycle.

The present solution proposes to create a multi-modal "clapper board" for example with an image-guided therapy system, injecting a synchronized timestamp in multiple modalities, functioning as the t=0 for synchronization purposes of the data of the different modalities. For some modalities, hardware may need to be modified, but for most a detectable signal can be generated by the multi-domain signal generator.

According to an aspect, a multi-modal synchronizer event e.g. "clapper board" is provided. A synchronized timestamp is injected in multiple modalities, e.g. functioning as a t=0 event that makes it easy to synchronize modalities.

In an option, a timestamping pulse is injected at the very end of the recording. This may help when modalities have unknown but fixed sampling rates or (linear) drift, e.g. to match timing from beginning to end of the recording.

In an option, more than a single detectable spike is injected in each of the modalities. Additionally, a binary signal is continuously encoded over the modalities, building up to an encoded timestamp, e.g. starting at 0 at the initial pulse. This may help when modalities have varying sampling rates, leading to non-linear drift. This will require a continuous overlaid signal in all modalities and may interfere with image quality.

The invention can be used in any type of imaging procedure where multiple modalities are used, such as minimally invasive interventional procedures and surgical procedures.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a multi-domain signal generator for temporal alignment of medical data acquisition in a multi-modality arrangement.
Fig. 2 shows another example of the multi-domain signal generator of Fig. 1.
Fig. 3 shows a further example of the multi-domain signal generator of Fig. 1.
Fig. 4 shows an example of a multi-modality medical data acquisition system.
Fig. 5 shows another example of a multi-modality medical data acquisition system.
Fig. 6 shows basic steps of an example of a method for temporal alignment of medical data acquisition in a multi-modality arrangement.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

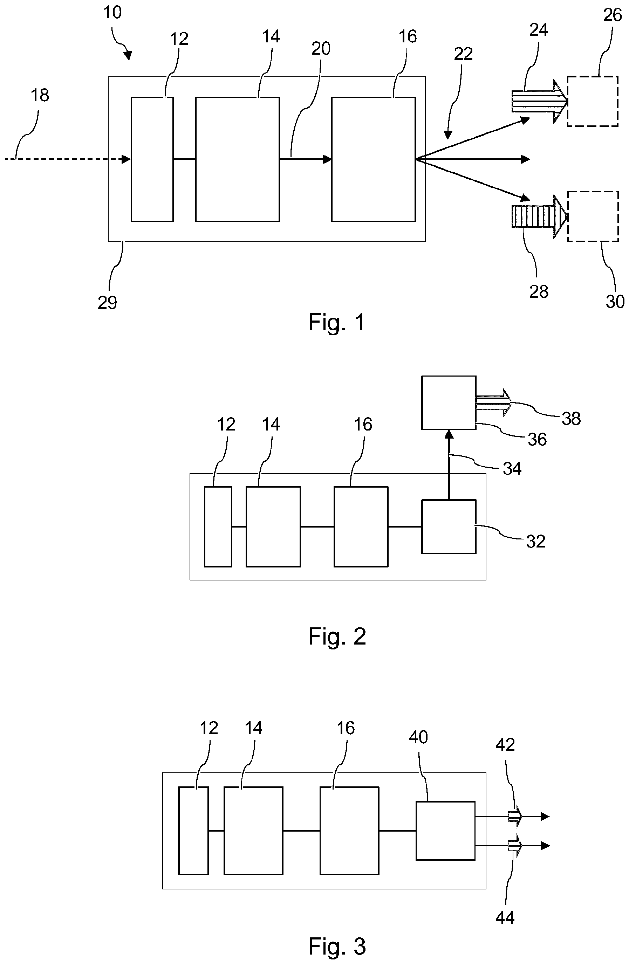

Fig. 1 schematically shows an example of a multi-domain signal generator 10 for temporal alignment of medical data acquisition in a multi-modality arrangement. The generator 10 comprises an activation interface 12, a control circuit 14 and a timestamping provider 16. The activation interface 12 is configured to receive an input signal 18 for a temporal alignment of the multi-modality arrangement and to transmit the input signal 18 to the control circuit 14. The control circuit 14 is configured to provide a trigger signal 20 for an activation of the timestamping provider 16 upon receiving the input signal 18. The timestamping provider 16 is configured, upon receiving the trigger signal 18, to activate a generation of a predetermined multi-modality timestamp pulse 22. The timestamp pulse 22 causes a modulation of at least one physical parameter of a first measuring signal 24 receivable by a first medical data acquisition modality 26 and a modulation of at least one physical parameter of a second measuring signal 28 receivable by a second medical data acquisition modality 30. The first measuring signal 24 and the second measuring signal 28 are from different medical data acquisition modalities.

The activation interface 12, the control circuit 14 and the timestamping provider 16 can be arranged in a common housing 29 or can be arranged as separate components or units.

The multi-domain signal generator 10 relates to generating a timestamping signal in at least two different data acquisition schemes. The multi-domain signal generator 10 can also be referred to as multidimensional signal generator.

The timestamp pulse 22 is receivable in at least a first and a second medical data acquisition domain where medical data is acquired at least in a first and a second signal domain being different from another.

The timestamp pulse 22 is receivable by at least the first medical data acquisition modality 26 and the second medical data acquisition modality 30. The first medical data acquisition 26 modality acquires medical data with a first data acquisition technique, and the second medical data acquisition modality 30 acquires medical data with a second data acquisition technique. The second data acquisition technique is different from the second data acquisition technique.

The multi-modality arrangement can also be referred to as multi-modality setup.

In an example, the timestamping provider 16 is a multi-domain pulse signal generator.

The timestamping provider 16 transfers a uniform signal into different domain signals.

The measuring signals are provided for an interaction with an object of interest, i.e. a region of interest of a subject. The interaction is detected by a receiver or detector. The timestamp pulse 22 is added to the respective measuring signal such that a change is detectable by the receiver or detector. The timestamp pulse 22 provides a modification that is detectable at the measuring signal receiver or measuring signal detector.

In an option, the timestamp pulse 22 is provided to modify at least one control parameter that has an influence on the measuring signal of the at least two different data acquisition modalities.

Fig. 2 shows another example of the multi-domain signal generator of Fig. 1. The timestamping provider 16 comprises at least one interface 32 for providing a data connection 34 with a signal generator 36 that provides at least one of the first and second measuring signal 38, indicated with an arrow. The timestamp pulse 22 leads to a modification of the measuring signal 38 provided by the signal generator 36.

In an example, the interface 32 is provided for a data connection with a first signal generator that provides the first measuring signal. The timestamp pulse 22 is provided to the first signal generator to modify the first measuring signal generated by the first signal generator. Further, the timestamping provider comprises a pulse generator that provides a signal pulse in a domain of the second measuring signal.

In an example, not shown in detail, the timestamping provider 16 comprises a first interface for a data connection with the first signal generator that provides the first measuring signal, and a second interface for a data connection with the second signal generator that provides the second measuring signal. The timestamp pulse is provided to the first signal generator to modify the first measuring signal generated by the first signal generator and to the second signal generator to modify the second measuring signal generated by the second signal generator.

Fig. 3 shows a further example of the multi-domain signal generator of Fig. 1. The timestamping provider 10 comprises at least one pulse generator 40 that provides a first signal pulse 42 in a domain of the first measuring signal, and a second signal pulse 44 in a domain of the second measuring signal.

In one example, one pulse generator is provided that provides the first signal pulse in the domain of the first measuring signal and the second signal pulse in the domain of the second measuring signal.

In another example, a first pulse generator is provided that provides the first signal pulse in the domain of the first measuring signal, and a second pulse generator is provided that provides the second signal pulse in the domain of the second measuring signal.

In an option, the first measuring signal and the second measuring signal are from different signal domains.

In an option, the first measuring signal and the second measuring signal are from the same signal domains.

In another option, three or more measuring signals are provided that are from different signal domains.

In a further option, three or more measuring signals are provided that are partly from the same and partly from different signal domains.

In an example, the multi-modality arrangement comprises a plurality of different medical data acquisition modalities using measuring signals. The timestamp pulse 22 causes a modulation of at least one physical parameter of each of the measuring signals receivable by the respective medical data acquisition modality.

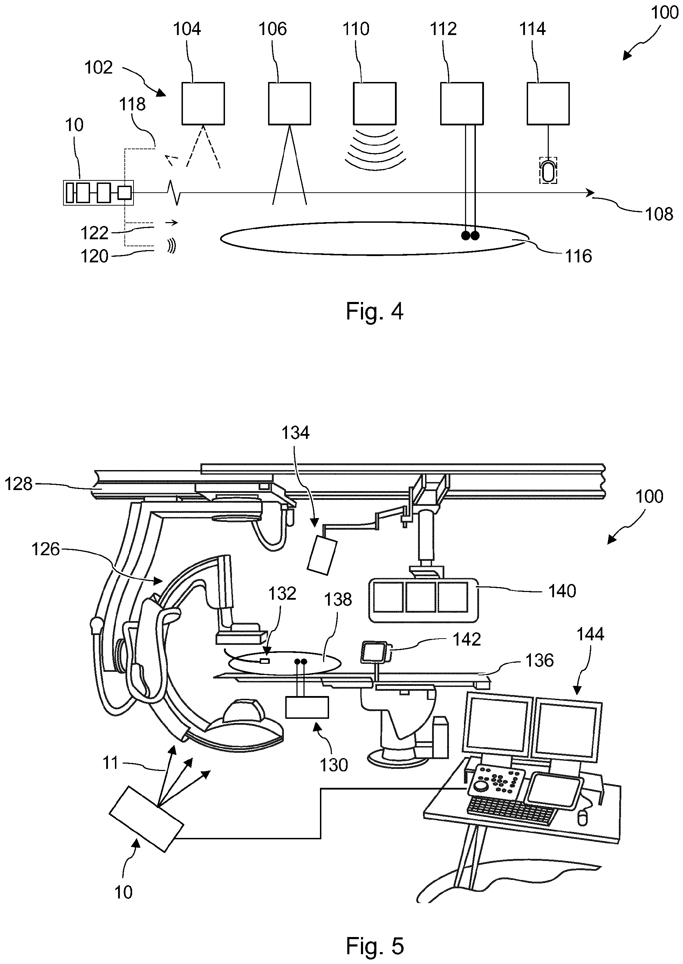

Fig. 4 schematically shows an example of a multi-modality medical data acquisition system 100. The system 100 comprises a plurality of data acquisition modalities 102 comprising at least a first medical data acquisition modality 104 applying a first measuring signal to provide a first data output and a second medical data acquisition modality 106 applying a second measuring signal to provide a second data output. The first measuring signal and the second measuring signal are different from each other. The system 100 further comprises an example of the multi-domain signal generator 10 according to one of the preceding examples. The multi-domain signal generator 10 generates a signal 108 that is received by several data acquisition modalities as a timestamp for temporal alignment.

The signal 108 is illustrated as an arrow across the different data acquisition fields of the different modalities to indicate that the signal 108 affects, i.e. reaches into the respective domains. It is noted that the signal 108 is shown as a single arrow, while the signal 108 represents an interaction with more than one type of measuring signal. Briefly said, the signal 108 comprises different signals or pulses for different domains.

As an option, further modalities are indicated. For example, a third medical data acquisition modality 110, a fourth medical data acquisition modality 112 and a fifth medical data acquisition modality 114 are shown. Further, a simplified subject 116 of interest to which the acquired data may relate to, is indicated.

In an option, the timestamp pulse 22 causes i) a modulation of at least one physical parameter of the first measuring signal received by the first medical data acquisition modality, and ii) a modulation of at least one physical parameter of the second measuring signal received by the second medical data acquisition modality.

In an option, the modulation of the at least one physical parameter of the first measuring signal provides a first timestamp signal as part of the first data output. The modulation of the at least one physical parameter of the second measuring signal provides a second timestamp signal as part of the second data output.

The data output is injected with the respective timestamp pulse.

In an option, further medical data acquisition modalities are provided applying measuring signals to provide a plurality of data outputs. The timestamp pulse 22 causes a modulation of at least one physical parameter of each of the measuring signals received by the further medical data acquisition modalities.

In an option, one of the data acquisition modalities, for example the first medical data acquisition modality 104, is an X-ray imaging modality comprising an X-ray source and an X-ray detector. The multi-domain signal generator comprises an X-ray source 118. The multi-domain signal generator 10 is arranged near the X-ray detector of the X-ray imaging modality.

As an example, the X-ray source of the multi-domain signal generator comprises at least one X-ray nano tube.

In another option, one of the data acquisition modalities, for example the third medical data acquisition modality 110, is an ultrasound imaging modality. The multi-domain signal generator 10 comprises a sound source 120 to generate a sound signal receivable by the ultrasound imaging modality.

In a further option, one of the data acquisition modalities, for example the second medical data acquisition modality 106, is an imaging modality based on light. The multi-domain signal generator comprises a light source 122 to generate a light signal receivable by the imaging modality.

In an option, one of the data acquisition modalities is provided as at least one of the group of:
- medical imaging comprising X-ray, CT, ultrasound, EM imaging or MRI;
- patient related data comprising hemodynamic, ECG and other vital data;
- device tracking comprising shape tracking;
- spatial and environmental imaging comprising time of flight, LIDAR;
- optical imaging comprising staff videos;
- acoustic recording comprising staff communication and procedure recording; and
- radiation tracking.

For example, the fourth medical data acquisition modality 112 is an example of a data acquisition modality providing patient related data comprising hemodynamic, ECG and other vital data.

For example, the fifth medical data acquisition modality 114 is an example of a data acquisition modality providing acoustic recording comprising staff communication and procedure recording.

In an option, based on the timestamp pulse 22, data acquired by the different data acquisition modalities is combinable in a temporally synchronized manner. As an option, the timestamp pulse 22 is provided in a resolution according to a smallest resolution of the different data acquisition modalities.

As an example, the timestamping system comprises at least one, for example two or more, of the group of audio pulse, X-ray pulse, optical pulse, ultrasound pulse and EM-pulse. In an example of the audio pulse, the system modulates a single spike or amplitude or frequency modulated spike, e.g. on top of the audio signal. As an option, the start time must be perfectly aligned with other signals. In an example of the X-ray pulse, an intensity peak in the X-ray signal is modulated. This can be a single peak or a more complex pattern. In an example of the optical pulse, the lasers in the detector or an operating lamp are used. In an option, an optical pulse is used that is too short for the human eye or to distract, or a wavelength is used that is not visible for the human eye but visible for instance for CMOS sensor. In an example of the EM-pulse, EM tracking coils are used. An electro-magnetic pulse is created, which can be tracked with the EM pickup sensor.

Apart from having them available to the physician to guide during the procedure, registered modalities and other data sources may also be used for other uses, such as work efficiency. For instance, (non-real-time) data mining may reveal the time spent in certain steps of the procedure and may reveal workflow bottlenecks and be used to create dashboards of key performance indicators. For data mining for workflow efficiency analysis, mutual temporal registration is important to extract meaningful performance indicators. Therefore, the present solution provides an improved and facilitated way to temporal register the various data coming from different modalities.

Fig. 5 shows another example of the multi-modality medical data acquisition system 100. The system comprises several medical data acquisition modalities. For example, an X-ray system 126 with an X-ray C-arm movably mounted to ceiling rails 128 is provided. Further, an ECG tracking device 130 is provided plus an ultrasound imaging device 132 like a catheter applied intravascular ultrasound system (IVUS). Still further, a camera 134 is arranged above a subject support 136 on which a subject 138 is schematically shown. Still further, a monitor arrangement 140 and a bedside controller 142 are shown. A console 144 is indicated in the foreground on the right side of Fig. 5.

The multi-domain signal generator 10 is shown separately in the lower left corner of the drawing of Fig. 5 to indicate its interaction with the different entities of the multi-modality arrangement. Three arrows 11 illustrate the respective interaction with the different detecting components recording the measuring signals. As explained above, this "reaching into" the respective domains of the timestamp pulse 22 can be provided in various ways. For some domains, like visible light cameras or acoustic recordings, a respective light or soundwave signal can be provided. For domains like X-ray, in an option, a respective radiation wave can be generated near the detector such that the radiation directly hits the detector without adding any extra dose to the subject of the staff. In another option, a respective radiation wave signal is generated within the X-ray tube used for X-ray imaging the subject.

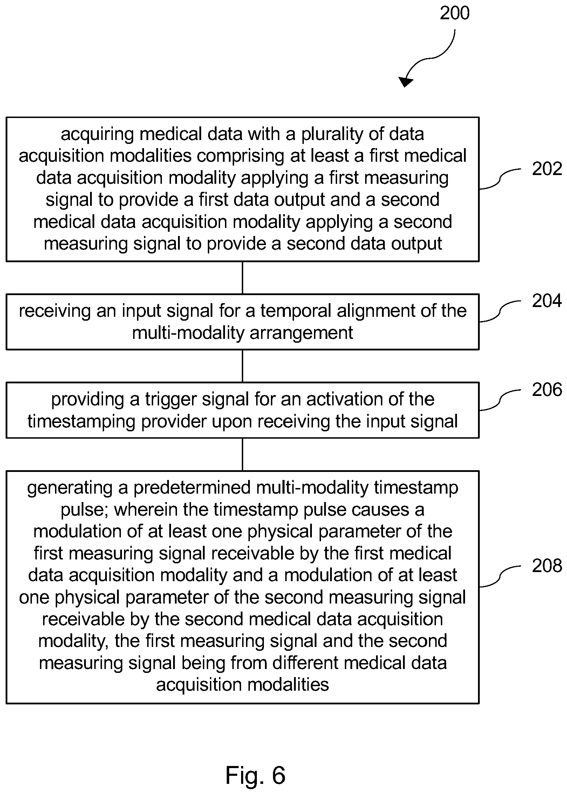

Fig. 6 shows basic steps of an example of a method 200 for temporal alignment of medical data acquisition in a multi-modality arrangement. The method 200 comprises the following steps:
- In a first step 202, medical data is acquired with a plurality of data acquisition modalities comprising at least a first medical data acquisition modality applying a first measuring signal to provide a first data output and a second medical data acquisition modality applying a second measuring signal to provide a second data output.
- In a second step 204, an input signal is received for a temporal alignment of the multi-modality arrangement.
- In a third step 206, a trigger signal is provided for an activation of the timestamping provider upon receiving the input signal.
- Further, in a fourth step 208, a predetermined multi-modality timestamp pulse is generated. The timestamp pulse causes a modulation of at least one physical parameter of the first measuring signal receivable by the first medical data acquisition modality and a modulation of at least one physical parameter of the second measuring signal receivable by the second medical data acquisition modality, the first measuring signal and the second measuring signal being from different medical data acquisition modalities.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A multi-domain signal generator (10) for temporal alignment of medical data acquisition in a multi-modality arrangement, the generator comprising:
- an activation interface (12);
- a control circuit (14); and
- a timestamping provider (16);
wherein the activation interface is configured to receive an input signal (18) for a temporal alignment of the multi-modality arrangement and to transmit the input signal to the control circuit;
wherein the control circuit is configured to provide a trigger signal (20) for an activation of the timestamping provider upon receiving the input signal; and
wherein the timestamping provider is configured, upon receiving the trigger signal, to activate a generation of a predetermined multi-modality timestamp pulse (22); wherein the timestamp pulse causes a modulation of at least one physical parameter of a first measuring signal (24) receivable by a first medical data acquisition modality (26) and a modulation of at least one physical parameter of a second measuring signal (28) receivable by a second medical data acquisition modality (30), the first measuring signal and the second measuring signal being from different medical data acquisition modalities.

2. Signal generator according to claim 1, wherein the timestamp pulse is provided to modify at least one control parameter that has an influence on the measuring signal of at least two different data acquisition modalities.

3. Signal generator according to claim 1 or 2, wherein the timestamping provider comprises at least one interface (32) for providing a data connection (34) with a signal generator (36) that provides at least one of the first and second measuring signal; and
wherein the timestamp pulse leads to a modification of the measuring signal provided by the signal generator.

4. Signal generator according to claim 1, 2 or 3,wherein the timestamping provider comprises at least one pulse generator (40) that provides a first signal pulse (42) in a domain of the first measuring signal, and a second signal pulse (44) in a domain of the second measuring signal.

5. Signal generator according to one of the preceding claims, wherein the first measuring signal and the second measuring signal are from different signal domains.

6. A multi-modality medical data acquisition system (100), the system comprising:
- a plurality of data acquisition modalities (102) comprising at least a first medical data acquisition modality (104) applying a first measuring signal to provide a first data output and a second medical data acquisition modality (106) applying a second measuring signal to provide a second data output; wherein the first measuring signal and the second measuring signal are different from each other; and
- a multi-domain signal generator (10) according to one of the preceding claims; wherein the multi-domain signal generator generates a signal that is received by several data acquisition modalities as a timestamp for temporal alignment.

7. System according to claim 6, wherein the timestamp pulse causes:
i) a modulation of at least one physical parameter of the first measuring signal received by the first medical data acquisition modality; and
ii) a modulation of at least one physical parameter of the second measuring signal received by the second medical data acquisition modality.

8. System according to claim 6 or 7, wherein the modulation of the at least one physical parameter of the first measuring signal provides a first timestamp signal as part of the first data output; and
wherein the modulation of the at least one physical parameter of the second measuring signal provides a second timestamp signal as part of the second data output.

9. System according to claim 6, 7 or 8, wherein further medical data acquisition modalities are provided applying measuring signals to provide a plurality of data outputs; and
wherein the timestamp pulse causes a modulation of at least one physical parameter of each of the measuring signals received by the further medical data acquisition modalities.

10. System according to one of the claims 6-9, wherein one of the data acquisition modalities is an X-ray imaging modality comprising an X-ray source and an X-ray detector;
wherein the multi-domain signal generator comprises an X-ray source (118); and wherein the multi-domain signal generator is arranged near the X-ray detector of the X-ray imaging modality.

11. System according to one of the claims 6-10, wherein one of the data acquisition modalities is an ultrasound imaging modality; and
wherein the multi-domain signal generator comprises a sound source (120) to generate a sound signal receivable by the ultrasound imaging modality.

12. System according to one of the claims 6-11, wherein one of the data acquisition modalities is an imaging modality based on light; and
wherein the multi-domain signal generator comprises a light source (122) to generate a light signal receivable by the ultrasound imaging modality.

13. System according to one of the claims 6-12, wherein one of the data acquisition modalities is provided as at least one of the group of:
- medical imaging comprising X-ray, CT, ultrasound, EM imaging or MRI;
- patient related data comprising hemodynamic, ECG and other vital data;
- device tracking comprising shape tracking;
- spatial and environmental imaging comprising time of flight, LIDAR;
- optical imaging comprising staff videos;
- acoustic recording comprising staff communication and procedure recording; and
- radiation tracking.

14. System according to one of the claims 6-13, wherein, based on the timestamp pulse, data acquired by the different data acquisition modalities is combinable in a temporally synchronized manner; and
wherein the timestamp pulse is provided in a resolution according to a smallest resolution of the different data acquisition modalities.

15. A method (200) for temporal alignment of medical data acquisition in a multi-modality arrangement, comprising the following steps:
- acquiring (202) medical data with a plurality of data acquisition modalities comprising at least a first medical data acquisition modality applying a first measuring signal to provide a first data output and a second medical data acquisition modality applying a second measuring signal to provide a second data output;
- receiving (204) an input signal for a temporal alignment of the multi-modality arrangement;
- providing (206) a trigger signal for an activation of the timestamping provider upon receiving the input signal; and
- generating (208) a predetermined multi-modality timestamp pulse; wherein the timestamp pulse causes a modulation of at least one physical parameter of the first measuring signal receivable by the first medical data acquisition modality and a modulation of at least one physical parameter of the second measuring signal receivable by the second medical data acquisition modality, the first measuring signal and the second measuring signal being from different medical data acquisition modalities.
